# FASCICULE DE BREVET EUROPEEN

(11) **EP 3 016 633 B1**
(45) Date de publication et mention de la délivrance du brevet: **14.03.2018**
(21) Numéro de dépôt: 14749908.1
(22) Date de dépôt: 04.07.2014
(51) Int. Cl.: A61K 8/365, A61K 8/37, A61K 8/60, A61K 8/73, A61Q 19/00, A61K 8/85, A61K 8/92, A61Q 19/08

(54) **COMPOSITION COSMÉTIQUE COMPRENANT UN CORPS GRAS PÂTEUX ET UN DÉRIVÉ NON IONIQUE DE CELLULOSE MODIFIÉ HYDROPHOBE**
KOSMETISCHE ZUBEREITUNG MIT PASTÖSER FETTIGER SUBSTANZ UND EINEM NICHTIONISCHEN DERIVAT VON CELLULOSE, DAS HYDROPHOB MODIFIZIERT IST
COSMETIC COMPOSITION COMPRISING A PASTY FATTY SUBSTANCE AND A NON-IONIC DERIVATIVE OF CELLULOSE WHICH IS HYDROPHOBICALLY MODIFIED

(30) Priorité: 04.07.2013 FR 1356543; 04.07.2013 FR 1356541
(43) Date de publication de la demande: 11.05.2016
(60) Demande divisionnaire: 17164826.4
(73) Titulaire: L'Oréal, 75008 Paris (FR)
(72) Inventeur: LORANT, Raluca, F-94320 Thiais (FR); CHABRILLANGEAS, Mathieu, F-75012 Paris (FR); BOILEAU, Nathalie, F-91070 Bondoufle (FR); EYRAUD, Sonia, F-94700 Maison-Alfort (FR)
(74) Mandataire: Prevel, Estelle Nicole
(86) Numéro de dépôt international: PCT/FR2014/051721
(87) Numéro de publication internationale: WO 2015/001270

(56) Documents cités:
- EP-A1- 1 752 135
- WO-A2-2007/017196
- DATABASE GNPD [Online] MINTEL; novembre 2013 (2013-11), "Night Cream-Mask Repair Booster", XP002722775, Database accession no. 2180239

## Description

L'invention concerne une composition de type émulsion, en particulier une composition cosmétique destinée aux matières kératiniques, notamment à la peau et aux lèvres, aux cheveux et aux ongles. L'invention concerne également l'utilisation de la dite composition dans le domaine cosmétique ou dermatologique, et en particulier pour le soin, l'hygiène, la protection et / ou le maquillage de la peau du corps ou du visage, ou pour le soin des cheveux.

Dans le domaine cosmétique, et plus particulièrement dans le domaine du soin de la peau, du maquillage et de la photo-protection, il est courant d'utiliser des architectures galéniques comprenant une phase grasse contenant des corps gras solides. La plupart du temps ces galéniques sont anhydres (par exemple les rouges à lèvres) et plus rarement émulsionnées. Dans ce dernier cas, le taux de corps gras solides est assez faible (< 3 %), principalement pour des raisons de stabilité des émulsions et de sensorialité (apport d'effet poisseux, collant et gras).

L'utilisation des corps gras pâteux dans des émulsions est cependant particulièrement intéressante. En effet, ils permettent d'apporter des effets de nutrition, de confort et de rémanence intéressants pour traiter la peau (en particulier les peaux sèches) tout en apportant un meilleur agrément sensoriel par rapport aux compositions anhydres. En effet, contrairement aux produits anhydres qui sont gras, collants et manquent de fraîcheur, les émulsions contenant des corps gras pâteux sont nutritives et présentent un bien meilleur agrément sensoriel.

Les émulsions contenant des corps gras pâteux sont difficiles à stabiliser. Pour y parvenir, l'homme de l'art a souvent recours à l'utilisation de forts taux de tensioactifs émulsionnants. Ces derniers sont reconnus pour leur efficacité stabilisante mais posent souvent des problèmes d'inconfort, d'innocuité et / ou d'agrément sensoriel.

Ainsi, l'introduction des corps gras pâteux dans une émulsion, en particulier l'introduction de forts taux de corps gras pâteux, entraîne une altération de la stabilité de l'émulsion très rapidement, particulièrement des émulsions sans, ou avec un faible taux, de tensioactif émulsionnant.

Il subsiste donc le besoin de réaliser des compositions présentant à la fois :
▪ Des corps gras pâteux pour leurs bénéfices intrinsèques ;
▪ Une bonne sensorialité même lorsque le taux de corps gras pâteux est important (>3%) ;
▪ Une bonne stabilité des émulsions même lorsque le taux de tensioactif émulsionnant est faible ou lorsque l'émulsion est exempte de tensioactif émulsionnant.

Par ailleurs, dans le domaine de la formulation des soins anti-âge, un des enjeux majeurs est d'introduire dans des émulsions des actifs biologiques performants tout en apportant (ou en gardant) un bon niveau de sensorialité et d'agrément cosmétique afin d'inciter à l'utilisation de ces produits et ainsi intensifier leurs effets constatées sur la peau grâce à une utilisation régulière et répétée.

Les actifs choisis parmi les dérivés C-glucoside, en particulier le C-béta-D-xylopyranoside-2-hydroxy-propane, et les actifs choisis parmi les dérivés d'acide cucurbique, tels que le sel de sodium de l'acide 3-hydroxy-2-pentylcyclopentyl)acétique, sont des molécules actives particulièrement performantes pour traiter les problématiques du vieillissement de la peau.

Pour l'obtention de hauts niveaux de performance, ces actifs peuvent être utilisés à des forts taux (par exemple à des taux de 3-10 % en matière active de C-béta-D-xylopyranoside-2-hydroxy-propane et de 1-5 % en matière active de sel de sodium de l'acide 3-hydroxy-2-pentylcyclopentylacétique).

Ces actifs particulièrement efficaces peuvent cependant poser des problèmes de sensorialité telle que l'apport d'un effet collant et / ou rêche sur la peau et l'apparition de peluches pendant la phase d'application et / ou après pénétration du produit dans la peau. Ces désagréments sont certainement accentués par la présence des glycols utilisés comme « solvant » de la molécule active.

Un autre défi pour le formulateur des produits de soin anti-âge est de proposer des textures agréables et adéquates en termes de sensorialité pour la cible consommateurs. En effet, les consommateurs susceptibles d'utiliser des soins anti-âge ont des problématiques et des besoins sensoriels spécifiques pour leur peau, comme par exemple un besoin de textures riches, nutritives, apaisantes, hydratantes.

Enfin, l'introduction de ce type d'actifs dans des galéniques émulsionnées provoque des problèmes de déstabilisation des émulsions, cette instabilité pouvant aller jusqu'à un déphasage de l'émulsion.

Il subsiste donc le besoin de proposer des compositions pour le soin de la peau :
▪ enrichies en molécules actives ;
▪ stables ;
▪ apportant un bon niveau d'agrément sensoriel.

La demanderesse a constaté, de façon surprenante, que l'association d'un corps gras pâteux particulier et d'au moins un gélifiant cellulosique modifié hydrophobe, permettait d'obtenir des émulsions stables même sans l'utilisation de tensioactif émulsionnant ou avec un faible taux de tensioactif émulsionnant (par exemple inférieur à 3%), et apportait un bon agrément sensoriel. En particulier, lorsque le taux de corps gras pâteux est élevé, cette association permet d'éviter les inconvénients des produits connus comportant de forts taux de corps gras pâteux, tels que par exemple un effet gras ou collant sur la peau.

Elle a également constaté, de façon surprenante, que la formulation d'actifs choisis parmi les dérivés C-glucoside et les dérivés d'acide cucurbique dans une émulsion comprenant l'association d'un corps gras pâteux et d'au moins un gélifiant cellulosique modifié hydrophobe, permettait d'obtenir des émulsions stables même sans l'utilisation de tensioactif émulsionnant ou avec un faible taux de tensioactif émulsionnant (par exemple inférieur à 3%), et apportait un bon agrément sensoriel sans affecter l'activité anti-âge des actifs ainsi formulés. En particulier, même lorsque le taux d'actifs dans l'émulsion est élevé, la composition ne comporte pas les inconvénients des produits connus comportant de forts taux d'actifs, comme par exemple des problèmes de sensorialité telle que l'apport d'un effet collant et / ou rêche sur la peau et l'apparition de peluches pendant la phase d'application et / ou après pénétration du produit dans la peau.

Ainsi, la présente invention a pour premier objet une composition cosmétique sous forme d'émulsion comprenant au moins un polyester résultant de l'estérification, par un acide polycarboxylique, d'un ester d'acide hydroxy carboxylique aliphatique comprenant au moins deux groupes hydroxyle et au moins un dérivé non ionique de cellulose comprenant un ou plusieurs substituants hydrophobes ayant de 8 à 30 atomes de carbone.

Les compositions de l'invention étant destinées à une application topique sur la peau ou les phanères, elles comprennent un milieu physiologiquement acceptable, c'est-à-dire un milieu compatible avec toutes les matières kératiniques telles que la peau, les ongles, les muqueuses et les fibres kératiniques (telles que les cheveux, les cils).

La présente invention permet d'obtenir des émulsions contenant des corps gras pâteux qui sont stables, même sans l'utilisation de tensioactif émulsionnant ou avec un faible taux de tensioactif émulsionnant (par exemple inférieur à 3 %), et qui apportent un bon agrément sensoriel, même lorsque la composition comporte un fort taux de corps gras pâteux.

Ainsi, il est possible d'introduire dans la composition conforme à l'invention de forts taux de corps gras pâteux sans altérer sa stabilité afin d'obtenir par exemple une composition pour le soin des peaux sèches qui permet de combler le manque de lipides cutanés des peaux déshydratées, apporter confort et nutrition rémanente grâce à de bonnes propriétés filmogène, tout en ayant de bonnes propriétés sensorielles, par exemple un effet non gras et non collant et un aspect mat de la peau, ainsi que de bonnes propriétés de pénétration dans la peau.

La présente invention permet également d'obtenir des émulsions contenant des actifs choisis parmi les dérivés C-glucoside et les dérivés d'acide cucurbique qui sont stables, même sans l'utilisation de tensioactif émulsionnant ou avec un faible taux de tensioactif émulsionnant (par exemple inférieur à 3 %), et qui apportent un bon agrément sensoriel, même lorsque la composition comporte un fort taux de ces actifs. La composition selon l'invention apporte une sensorialité excellente, sans effet rêche ni gras ni collant et sans effet de pelluches.

De plus, elle permet de renforcer la perception d'efficacité grâce à des effets immédiats ressentis dès l'application du produit : effets nutritifs, apaisants, hydratants.

Ainsi, il est possible d'introduire dans la composition conforme à l'invention de forts taux d'actifs choisis parmi les dérivés C-glucoside et les dérivés d'acide cucurbique sans altérer sa stabilité, tout en ayant de bonnes propriétés sensorielles, par exemple un effet non collant et non rêche sans apparition de peluches à l'application et après pénétration du produit dans la peau, afin d'obtenir par exemple une composition pour le soin anti-âge des matières kératiniques, et en particulier de la peau, ayant de bonnes performances.

Par ailleurs, lorsque la composition comprend une ou plusieurs charges à effet optique et / ou un ou plusieurs pigments, elle apporte des propriétés matifiantes et soft focus sur la peau même lorsque le taux de corps gras pâteux et/ou lorsque le taux d'actifs dans la composition est élevé. De plus, la composition selon l'invention présente l'avantage de ne pas provoquer de peluches lors de son application sur la peau et / ou après sa pénétration dans la peau. Globalement, les propriétés d'usage sont améliorées.

La présente invention a également pour objet un procédé cosmétique de maquillage et /ou de soin des matières kératiniques comprenant une étape d'application d'une composition telle que définie plus haut sur lesdites matières kératiniques.

Dans ce qui va suivre, l'expression *« au moins un(e)* » est équivalente à « un(e) ou plusieurs » et, à moins d'une autre indication, les bornes d'un domaine de valeurs sont comprises dans ce domaine.

### Polyesters

La composition selon le premier objet de l'invention comprend au moins un polyester résultant de l'estérification, par un acide polycarboxylique, d'un ester d'acide hydroxy carboxylique aliphatique comprenant au moins deux groupes hydroxyle.

Le polyester selon l'invention est un copolymère qui résulte de l'estérification, par un acide polycarboxylique d'un ester d'acide hydroxy carboxylique aliphatique (que l'on appellera par la suite «ester hydroxylé»).

Le polyester selon l'invention a de préférence un poids moléculaire compris entre 3000 et 7000 g/mol. Par exemple, le Risocast DA-L a une masse moléculaire en nombre comprise entre 3500 et 4000 g/mol et le Risocast DA-H a une masse moléculaire en nombre comprise entre 5000 et 6500 g/mol. Ces produits sont commercialisés par la société japonaise KOKYU ALCOHOL KOGYO.

Le ratio molaire entre l'acide polycarboxylique et l'ester hydroxylé utilisés pour préparer le polyester selon l'invention est de préférence compris entre 0,25 et 1. Par exemple, ce ratio est égal à 0,75 pour le Risocast DA-H, et ce ratio est égal à 0,5 pour le Risocast DA-L.

On peut citer en particulier, comme polyester utilisable dans la composition selon l'invention :
- l'ester résultant de la réaction d'estérification de l'huile de ricin hydrogénée avec l'acide dilinoléïque dans les proportions 2 pour 1,
- l'ester résultant de la réaction d'estérification de l'huile de ricin hydrogénée avec l'acide isostéarique dans les proportions 4 pour 3.

Le polyester de la présente invention est avantageusement un composé pâteux ou visqueux à température ambiante (25°C). Par "pâteux" au sens de la présente invention, on entend désigner un composé gras lipophile, à changement d'état solide/liquide réversible, et comportant à la température de 23°C une fraction liquide et une fraction solide.

Par composé pâteux au sens de l'invention, on entend un composé ayant de préférence une dureté à 20 °C allant de 0,001 à 0,5 MPa, de préférence de 0,002 à 0,4 MPa.

A titre d'exemple, le Risocast DA-L a une dureté à 20 °C de 0,04 MPa, une fraction liquide à 23 °C égale à 82 %, et une fraction liquide à 32 °C égale à 90 %.

Le polyester selon l'invention résulte de l'estérification :
- d'un acide polycarboxylique, et
- d'un ester d'acide hydroxy carboxylique aliphatique (que l'on appellera par la suite «ester hydroxylé»).

### ESTER HYDROXYLE

L'ester d'acide hydroxy carboxylique aliphatique (ou ester hydroxylé) comprend au moins deux groupes hydroxyle.

L'ester hydroxylé est avantageusement issu de la réaction d'au moins un acide carboxylique aliphatique hydroxylé avec un polyol.

Ledit acide aliphatique hydroxylé comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptibles d'être estérifiés par la suite par l'acide polycarboxylique pour obtenir le polyester de la présente invention.

Ledit polyol peut comprendre de 2 à 40 atomes de carbone et mieux de 3 à 30 atomes de carbone. Le polyol est, de préférence, un polyol aliphatique. Avantageusement, le polyol n'est pas un ose.

Ledit polyol qui réagit avec l'acide hydroxylé décrit précédemment peut être partiellement ou totalement estérifié ; de façon avantageuse, le polyol est totalement estérifié.

De préférence, l'ester d'acide hydroxy carboxylique aliphatique est un ester d'acide gras hydroxylé tel que le reste acide gras comporte au moins 12 atomes de carbone, par exemple de 12 à 40 atomes de carbone, et mieux de 12 à 28 atomes de carbone.

L'ester d'acide hydroxy carboxylique aliphatique utilisable dans l'invention peut être choisi parmi :
a) les esters partiels ou totaux de monoacides carboxyliques aliphatiques mono hydroxylés linéaires saturés ;
b) les esters partiels ou totaux de monoacides carboxyliques aliphatiques mono hydroxylés insaturés tels que le triricinoléate de glycéryle (huile de ricin) ;
c) les esters partiels ou totaux de polyol aliphatique en C₂ à C₁₆ ayant réagi avec un mono ou un poly acide carboxylique aliphatique mono ou poly hydroxylé comme notamment les triglycérides, les esters du pentaérythritol, du triméthylol propane, du propylène glycol, du néopentyl glycol, du dipentaérythritol, du polyglycérol, les esters du sorbitol ;
   et leurs mélanges.

Avantageusement lorsque l'ester d'acide hydroxy carboxylique aliphatique résulte de l'estérification d'un polyacide carboxylique aliphatique tels que ceux cités ci-dessus, il ne reste pas de groupement COOH résiduel non engagé dans une liaison ester.
L'ester d'acide hydroxy carboxylique aliphatique est de préférence choisi parmi les esters de polyols aliphatiques en C₂ à C₁₆, lesdits polyols ayant réagi avec un acide gras aliphatique hydroxylé à chaîne saturée ou insaturée, comportant au moins 12 atomes de carbone. L'acide gras est de préférence l'acide ricinoléique et l'ester d'acide hydroxy carboxylique aliphatique est de préférence l'huile de ricin hydrogénée.

### ACIDE POLYCARBOXYLIQUE

L'acide polycarboxylique comprend au moins deux groupes COOH. Il est avantageusement un dimère diacide d'acide(s) carboxylique(s) aliphatique(s) insaturé(s).
L'acide polycarboxylique selon l'invention est de préférence aliphatique; il est avantageusement un acide dicarboxylique aliphatique.
Selon un mode réalisation, l'acide polycarboxylique est un dimère diacide d'acide(s) gras insaturé(s), c'est-à-dire un dimère formé à partir d'au moins un acide gras insaturé, par exemple à partir d'un seul acide gras insaturé ou à partir de deux acides gras insaturés différents. L'acide gras est de préférence mono insaturé ou di insaturé. On entend par acide gras, un acide obtenu par hydrolyse de corps gras d'origine végétale ou animale.

Les dimères diacide d'acide(s) gras insaturé(s) ou encore dimère diacide sont classiquement obtenus par réaction de dimérisation intermoléculaire d'au moins un acide gras insaturé. De préférence, on dimérise un seul type d'acide gras insaturé.
Les dimères diacide d'acide(s) gras insaturé(s) sont notamment obtenus par la dimérisation d'un acide gras insaturé notamment en C₈ à C₃₄, notamment en C₁₂ à C₂₂, en particulier en C₁₆ à C₂₀, et plus particulièrement en C₁₈.

A titre représentatif de ces acides gras insaturés, on peut notamment citer l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.
Le dimère diacide est de préférence saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles doubles liaisons en simples liaisons.
Les dimères diacides d'acide(s) gras insaturé(s) préférés sont obtenus par dimérisation de l'acide linoléique, puis éventuellement par hydrogénation du dimère ainsi obtenu. La forme hydrogénée peut être partielle ou totale et notamment correspondre à la forme saturée plus stable à l'oxydation.
On trouve également dans le commerce des dimères diacides et notamment des diacides dilinoléiques dont la stabilité vis-à-vis de l'oxydation a été améliorée par hydrogénation des doubles liaisons restant après la réaction de dimérisation.
Dans la présente invention, on peut utiliser tout dimère diacide disponible actuellement dans le commerce.

Le ou les polyesters peuvent être présents dans la composition conforme à l'invention en quantité en matière active comprise entre 0,1 % et 30 % en poids, de préférence entre 1 % et 10 %, et encore plus préférentiellement entre 2 % et 8 %en poids du poids total de la composition.
Selon un mode de réalisation particulier, la composition comprend le ou les polyesters tels que définis précédemment en une quantité en matière active au moins égale à 10 % en poids, de préférence entre 10 % et 50 % en poids, et encore plus préférentiellement entre 15 et 30 % en poids du poids total de la phase grasse.

### Dérivés non ioniques de cellulose comprenant un ou plusieurs substituants hydrophobes

Les compositions conformes à l'invention comprennent au moins un dérivé non ionique de cellulose comprenant un ou plusieurs substituants hydrophobes ayant de 8 à 30 atomes de carbone.
Par « dérivé de cellulose », on entend au sens de la présente invention un composé comportant au moins un motif cellobiose de structure suivante : 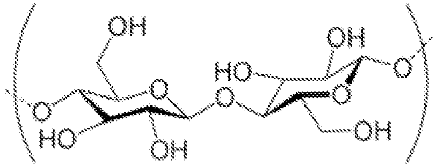 dans laquelle, un ou plusieurs groupes hydroxyle peuvent être substitués.
Le ou les dérivés non ioniques de cellulose à substituant(s) hydrophobe(s) conformes à la présente invention sont des polymères amphiphiles présentant un caractère associatif. En effet, ils comprennent des motifs hydrophiles et des motifs hydrophobes et sont capables d'interagir et de s'associer entre eux ou à d'autres molécules, de manière réversible, en particulier, grâce à la présence de leurs chaînes hydrophobes.
Selon un mode de réalisation particulier, le dérivé de cellulose de l'invention est un éther de cellulose comprenant un ou plusieurs substituants hydrophobes comprenant de 8 à 30 atomes de carbone.
Le ou les dérivés non ioniques de cellulose à substituant(s) hydrophobe(s) conformes à la présente invention sont préparés généralement à partir d'éthers non ioniques de cellulose hydrosolubles, dont on substitue tout ou partie des fonctions hydroxyle réactives par une ou plusieurs chaînes hydrophobes comprenant de 8 à 30 atomes de carbone, de préférence de 10 à 22 atomes de carbone, et mieux encore 16 atomes de carbone. Les étapes de réactions en jeu dans la préparation des dérivés de cellulose de l'invention sont connues de l'homme du métier.
Les éthers non ioniques de cellulose choisis pour préparer les dérivés non ioniques de cellulose à substituant(s) hydrophobe(s) selon l'invention ont de préférence un degré de substitution non ionique, par exemple en groupements méthyle, hydroxyéthyle ou hydroxypropyle, suffisant pour être hydrosolubles, c'est-à-dire former une solution sensiblement limpide lorsqu'ils sont dissous dans l'eau à 25 °C à la concentration de 1 % en poids.
Les éthers non ioniques de cellulose choisis pour préparer les dérivés non ioniques de cellulose à substituant(s) hydrophobe(s) selon l'invention possèdent préférentiellement une masse molaire moyenne en nombre relativement faible, inférieure à 800 000 g/mole, de préférence allant de 50 000 et 700 000 g/mole et de manière plus préférée allant de 200 000 à 600 000 g/mole.
De préférence, le dérivé de cellulose de l'invention est une hydroxyéthylcellulose comprenant un ou plusieurs substituants hydrophobes comprenant de 8 à 30 atomes de carbone.
Les dérivés non ioniques de cellulose utilisés selon l'invention sont substitués par une ou plusieurs chaînes hydrocarbonées en C₈-C₃₀ linéaires, ramifiées ou cycliques, saturées ou insaturées, aliphatiques ou aromatiques, pouvant être rattachées au substrat éther de cellulose par une liaison éther, ester, ou uréthane, de préférence éther.
Selon un mode de réalisation, le ou les substituants hydrophobes utilisés comme substituants des dérivés non ioniques de cellulose selon la présente invention sont des groupes alkyle, arylalkyle ou alkylaryle en C₈-C₃₀, de préférence en C₁₀-C₂₂.
De préférence, le ou les substituants hydrophobes selon la présente invention sont des chaînes alkyles saturées.
Selon un mode de réalisation préféré, le ou les substituants hydrophobes selon la présente invention sont des groupements cétyle.
Les dérivés non ioniques de cellulose à substituant(s) hydrophobe(s) selon l'invention présentent une viscosité de préférence comprise entre 100 et 100 000 mPa.s, et de préférence entre 200 et 20 000 mPa.s, mesurée à 25 °C dans une solution à 1 % en poids de polymère dans l'eau, cette viscosité étant déterminée de manière conventionnelle à l'aide d'un viscosimètre de type Brookfield LVT à 6 tours par minute avec le mobile n° 3.
Le degré de substitution hydrophobe des dérivés non ioniques de cellulose hydrophiles utilisés selon l'invention va préférentiellement de 0,1 à 10 % en poids, plus préférentiellement de 0,1 à 1 % en poids et de manière particulièrement préférée de 0,4 à 0,8 % en poids du poids total du polymère.
Parmi les dérivés non ioniques de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société ASHLAND.
Selon un mode de réalisation particulier, la concentration en matière active des dérivés non ioniques de cellulose à substituant(s) hydrophobe(s) dans la composition selon l'invention va de 0,05 à 20 % en poids, en particulier de 0,25 à 10 % en poids, et préférentiellement de 0,5 à 3 % en poids, par rapport au poids total de la composition.

La composition selon le deuxième objet de l'invention comprend au moins un actif choisi parmi les dérivés C-glucoside, en particulier le C-béta-D-xylopyranoside-2-hydroxy-propane, et les dérivés d'acide cucurbique, tels que le sel de sodium de l'acide 3-hydroxy-2-pentylcyclopentyl)acétique.

### Dérivés C-glycoside

Le ou les dérivés C-glycoside sont avantageusement dotés de la propriété d'exercer leurs effets hygroscopiques sur la peau et non dans la composition cosmétique. Avantageusement, une composition cosmétique comprenant un dérivé C-glycoside peut exercer une action de soin à long terme, même après son élimination des tissus.
Le ou les dérivés C-glycoside pouvant être présents dans la composition conforme à l'invention sont choisis parmi les composés de formule générale (I) suivante : 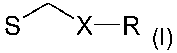 dans laquelle :
- R désigne un radical alkyle linéaire non substitué en C₁-C₄, notamment en C₁-C₂, en particulier le méthyle ;
- S représente un monosaccharide choisi parmi le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)- ;
ainsi que leurs sels cosmétiquement acceptables, leurs solvates tels que les hydrates et leurs isomères optiques.
A titre illustratif et non limitatif des dérivés C-glycoside convenant plus particulièrement à l'invention, on peut notamment citer les dérivés suivants :
- le C-béta-D-xylopyranoside-n-propane-2-one ;
- le C-alpha-D-xylopyranoside-n-propane-2-one ;
- le C-béta-D-xylopyranoside-2-hydroxy-propane ;
- le C-alpha- D-xylopyranoside-2-hydroxy-propane ;
- 1-(C-béta-D-glucopyranosyl)-2-hydroxy-propane ;
- 1-(C-alpha-D-glucopyranosyl)-2-hydroxy-propane ;
- 1-(C-béta-D-glucopyranosyl)-2-amino-propane ;
- 1-(C-alpha-D-glucopyranosyl)-2-amino-propane ;
- 3'-(acétamido-C-béta-D-glucopyranosyl)-propane-2'-one ;
- 3'-(acétamido-C-alpha-D-glucopyranosyl)-propane-2'-one ;
- 1-(acétamido-C-béta-D-glucopyranosyl)-2-hydroxyl-propane ;
- 1-(acétamido-C-béta-D-glucopyranosyl)-2-amino-propane ;
- ainsi que leurs sels cosmétiquement acceptables, leurs solvates tels que les hydrates et leurs isomères optiques..

Selon un mode de réalisation particulier, le C-béta-D-xylopyranoside-2-hydroxy-propane ou le C-alpha-D-xylopyranoside-2-hydroxy-propane, et mieux le C-béta-D-xylopyranoside-2-hydroxy-propane, peuvent être avantageusement mis en oeuvre pour la préparation d'une composition selon l'invention.
Selon un mode particulier de réalisation, un dérivé C-glycoside convenant à l'invention peut être avantageusement l'hydroxypropyl-tétrahydropyrantriol également dénommé C-béta-D-xylopyranoside-2-hydroxy-propane, notamment proposé en solution à 30 % en poids dans un mélange Eau/propylèneglycol (60/40) sous la dénomination MEXORYL SBB® par CHIMEX. Selon un mode de réalisation, le dérivé C-glycoside est sous la forme d'une solution dans laquelle il est présent en une quantité de 30% en poids par rapport au poids total de la solution, le reste étant un mélange d'eau et de propylène glycol.
Les sels des dérivés C-glycoside convenant à l'invention peuvent comprendre des sels physiologiquement acceptables conventionnels de ces composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique et l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.
Les solvates acceptables pour les composés décrits ci-dessus comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.
Un dérivé C-glycoside convenant à l'invention peut notamment être obtenu par la méthode de synthèse décrite dans le document WO 02/051828, dont le contenu est incorporé ici par référence.
Selon un mode de réalisation, la composition selon l'invention comprend un dérivé C-glycoside en quantité comprise entre 0,03 % et 30 % en poids de matière active (dérivé C-glycoside) par rapport au poids total de la composition, en particulier entre 0,03 % et 10 % en poids de matière active par rapport au poids total de la composition, plus particulièrement entre 0,05 % et 5 % en poids de matière active par rapport au poids total de la composition.

### Dérivés d'acide cucurbique

Le ou les dérivés d'acide cucurbique pouvant être présents dans la composition conforme à l'invention sont des composés choisis parmi ceux répondant à la formule (II) suivante : 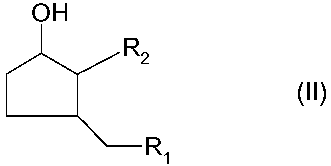 dans laquelle :
R₁ représente un radical COOR₃, R₃ désignant un atome d'hydrogène ou un radical alkyle en C₁-C₄, éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ;
ainsi que leurs sels cosmétiquement acceptables, leurs solvates tels que les hydrates et leurs isomères optiques.
De préférence, R₁ désigne un radical choisi parmi -COOH, -COOMe, -COO-CH₂-CH₃, - COO-CH₂-CH(OH)-CH₂OH, -COOCH₂-CH₂-CH₂OH, -COOCH₂-CH(OH)-CH₃. Préférentiellement, R₁ désigne un radical -COOH.
Préférentiellement, R₂ désigne un radical hydrocarboné, linéaire, saturé ou insaturé, et de préférence ayant de 2 à 7 atomes de carbone. En particulier, R₂ peut être un radical pentyl, pentenyl, hexyle, heptyle.
Selon un mode de réalisation, le dérivé d'acide cucurbique est choisi parmi l'acide 3-hydroxy-2-[(2Z)-2-pentenyl]-cyclopentane acétique ou l'acide 3-hydroxy-2-pentyl-cyclopentane acétique, ainsi que leurs sels cosmétiquement acceptables, leurs solvates tels que les hydrates et leurs isomères optiques. De préférence, le dérivé d'acide cucurbique est choisi parmi l'acide 3-hydroxy-2-pentyl-cyclopentane acétique, ainsi que ses sels cosmétiquement acceptables, ses solvates tels que les hydrates et ses isomères optiques.; ce composé peut être notamment sous la forme de sel de sodium. A titre d'exemple, on peut citer le sel de sodium de 3-hydroxy-2-pentyl-cyclopentane acétique à 30 % dans le mélange eau/dipropylène glycol 70/30 à pH neutre commercialisé sous la dénomination MEXORYL SBO ® par la société CHIMEX.
Les sels des composés utilisables selon l'invention sont en particulier choisis parmi les sels de métal alcalin, par exemple sodium, potassium ; les sels de métal alcalino-terreux, par exemple calcium, magnésium, strontium, les sels métalliques, par exemple zinc, aluminium, manganèse, cuivre ; les sels d'ammonium de formule NH₄⁺ ; les sels d'ammonium quaternaires ; les sels d'amines organiques, comme par exemple les sels de méthylamine, de diméthylamine, de triméthylamine, de triéthylamine, d'éthylamine, de 2-hydroxyéthylamine, de bis-(2-hydroxyéthyl)amine, de la tri-(2-hydroxyéthyl)amine ; les sels de lysine, d'arginine. On utilise de préférence les sels choisis parmi les sels de sodium, potassium, magnésium, strontium, cuivre, manganèse, zinc. Préférentiellement, on utilise le sel de sodium.
Les solvates acceptables pour les composés décrits ci-dessus comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.
Le ou les dérivés d'acide cucurbique tels que définis précédemment peuvent être présents dans la composition selon l'invention en une teneur en matière active allant de 0,01 à 15 % en poids par rapport au poids total de la composition, de préférence de 0,01 % à 12 % en poids, et plus particulièrement de 0,05 à 5 % en poids.

### Phase grasse

La proportion de la phase grasse peut aller par exemple de 0,5 à 60 % en poids, de préférence de 5 à 40%, et encore plus préférentiellement de 10 à 35 % en poids par rapport au poids total de la composition.
Cette quantité indiquée comprend la teneur en polyesters tels que définis précédemment lorsque la composition en contient. Par contre, elle ne comprend pas la teneur en tensioactifs lipophiles, lorsque la composition en contient.
Au sens de l'invention, la phase grasse inclut tout corps gras liquide à température ambiante et pression atmosphérique, généralement des huiles, ou solide à température ambiante et pression atmosphérique, à l'image des cires, ou tout composé pâteux, présents dans ladite composition, y compris les polyesters tels que définis précédemment.
La phase grasse des compositions conformes à l'invention comprend au moins un corps gras pâteux.
Par "corps gras pâteux" au sens de la présente invention, on entend un composé gras lipophile à changement d'état solide/liquide réversible, présentant à l'état solide une organisation cristalline anisotrope, et comportant à la température de 23 °C une fraction liquide et une fraction solide.
En d'autres termes, la température de fusion commençante du corps gras pâteux peut être inférieure à 23 °C. La fraction liquide du corps gras pâteux mesurée à 23 °C peut représenter 9 à 97 % en poids du corps gras pâteux. Cette fraction liquide à 23 °C représente de préférence entre 15 et 85 %, de préférence encore entre 40 et 85 % en poids.
Au sens de l'invention, la température de fusion correspond à la température du pic le plus endothermique observé en analyse thermique (DSC) telle que décrite dans la norme ISO 11357-3 ; 1999. Le point de fusion d'un corps gras pâteux peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de corps gras pâteux disposé dans un creuset est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de corps gras pâteux en fonction de la température. Le point de fusion du corps gras pâteux est la valeur de la température correspondant au sommet du pic de la courbe représentant la variation de la différence de puissance absorbée en fonction de la température.
La fraction liquide en poids du corps gras pâteux à 23 °C est égale au rapport de l'enthalpie de fusion consommée à 23 °C sur l'enthalpie de fusion du corps gras pâteux.
L'enthalpie de fusion du corps gras pâteux est l'enthalpie consommée par ce dernier pour passer de l'état solide à l'état liquide. Le corps gras pâteux est dit à l'état solide lorsque l'intégralité de sa masse est sous forme solide cristalline. Le corps gras pâteux est dit à l'état liquide lorsque l'intégralité de sa masse est sous forme liquide.
L'enthalpie de fusion du corps gras pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999.
L'enthalpie de fusion du corps gras pâteux est la quantité d'énergie nécessaire pour faire passer le corps gras pâteux de l'état solide à l'état liquide. Elle est exprimée en J/g.
L'enthalpie de fusion consommée à 23 °C est la quantité d'énergie absorbée par l'échantillon pour passer de l'état solide à l'état qu'il présente à 23 °C constitué d'une fraction liquide et d'une fraction solide.
La fraction liquide du corps gras pâteux mesurée à 32 °C représente de préférence de 30 à 100 % en poids du corps gras pâteux, de préférence de 50 à 100 %, de préférence encore de 60 à 100 % en poids du corps gras pâteux. Lorsque la fraction liquide du corps gras pâteux mesurée à 32 °C est égale à 100 %, la température de la fin de la plage de fusion du corps gras pâteux est inférieure ou égale à 32 °C.
La fraction liquide du corps gras pâteux mesurée à 32 °C est égale au rapport de l'enthalpie de fusion consommée à 32 °C sur l'enthalpie de fusion du corps gras pâteux. L'enthalpie de fusion consommée à 32 °C est calculée de la même façon que l'enthalpie de fusion consommée à 23 °C.

Le corps gras pâteux peut être choisi parmi les corps gras synthétiques et les corps gras d'origine végétale. Un corps gras pâteux peut être obtenu par synthèse à partir de produits de départ d'origine végétale.
Le corps gras pâteux est avantageusement choisi parmi :
- la lanoline et ses dérivés,
- les éthers de polyol choisi parmi les éthers de pentaérythritol et de polyalkylène glycol, les éthers d'alcool gras et de sucre, et leurs mélanges. l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société VEVY, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja,
- les composés siliconés polymères ou non,
- les composés fluorés polymères ou non,
- les polymères vinyliques, notamment :
   ▪ les homopolymères et les copolymères d'oléfines,
   ▪ les homopolymères et copolymères de diènes hydrogénés,
- les polyéthers liposolubles résultant de la polyéthérification entre un ou plusieurs diols en C2-C100, de préférence en C2-C50,
- les esters,
- et/ou leurs mélanges.
Le corps gras pâteux est de préférence un polymère, notamment hydrocarboné.
Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par AKZO NOBEL.
Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique, l'acide isostéarique et l'acide 12-hydroxystéarique, à l'image notamment de ceux commercialisé sous la marque Softisan 649 par la société SASOL,
- le propionate d'arachidyle commercialisé sous la marque Waxenol 801 par ALZO,
- les esters de phytostérol,
- les triglycérides d'acides gras et leurs dérivés,
- les esters de pentaérythritol,
- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate; de tels esters peuvent être notamment choisis parmi les esters de nomenclature INCI suivante : le bis-béhényl/isostéaryl/phytostéryl dimerdilinoléyle dimerdilinoléate (Plandool G), le phytostéryl isostéaryl dimerdilinoléate (Lusplan PI-DA, Lusplan PHY/IS-DA), le phytostéryl/isostéryl/cétyl/stéaryl/béhényl dimerdilinoléate (Plandool H ou Plandool S) et leurs mélanges.
On peut également citer comme autres corps gras pateux préférés :
- le beurre de mangue, tel que celui commercialisé sous la référence Lipex 203 par la société AARHUSKARLSHAMN,
- l'huile de soja hydrogénée, l'huile de coprah hydrogénée, l'huile de colza hydrogénée, les mélanges d'huiles végétales hydrogénées tels que le mélange d'huile végétale hydrogénée de soja, coprah, palme et colza, par exemple le mélange commercialisé sous la référence Akogel® par la société AARHUSKARLSHAMN (nom INCI Hydrogenated Vegetable Oil),
- le beurre de karité, en particulier celui dont le nom INCI est Butyrospermum Parkii Butter, tel que celui commercialisé sous la référence Sheasoft® par la société AARHUSKARLSHAMN,
- le beurre de cacao, en particulier celui qui est commercialisé sous la dénomination CT COCOA BUTTER DEODORIZED par la société DUTCH COCOA BV ou celui qui est commercialisé sous la dénomination BEURRE DE CACAO NCB HD703 758 par la société BARRY CALLEBAUT ;
- le beurre de shorea, en particulier celui qui est commercialisé sous la dénomination DUB SHOREA T par la société STEARINERIE DUBOIS ;
- et leurs mélanges.

Selon un mode de réalisation particulier de l'invention, la composition comprend au moins un corps gras pâteux choisi parmi les polyesters tels que définis précédemment.

La phase grasse des compositions conformes à l'invention peut en outre comprendre au moins un corps gras choisis parmi les corps gras solides tels que les cires et les corps gras liquides tels que les huiles.

Les cires considérées dans le cadre de la présente invention sont d'une manière générale des composés lipophiles, solides, déformables ou non déformables, à température ambiante (25 °C), à changement d'état solide/liquide réversible, ayant un point de fusion supérieur ou égal à 30 °C pouvant aller jusqu'à 200 °C et notamment jusqu'à 120 °C.
En portant une ou des cires, conforme(s) à l'invention, à l'état liquide (fusion), il est possible de la ou les rendre miscibles à une ou des huiles et de former un mélange cire(s) + huile(s), homogène macroscopiquement, mais en ramenant la température dudit mélange à la température ambiante, on obtient une recristallisation de la ou des cire(s) dans la ou les huile(s) du mélange.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire, disposé dans un creuset, est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin est soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.
Les cires susceptibles d'être utilisées dans une composition selon l'invention sont choisies parmi les cires, solides à température ambiante, d'origine animale, végétale, minérale ou de synthèse et leurs mélanges. Elles peuvent être hydrocarbonées, fluorées et/ou siliconées.
A tire d'exemples, on peut notamment citer les cires hydrocarbonées, comme la cire d'abeille naturelle (ou cire d'abeille blanchie), la cire d'abeille synthétique, la cire de Carnauba, la cire de son de riz telle que celle commercialisée sous la référence NC 1720 par la société CERA RICA NODA, la cire de Candellila telle que celle commercialisée sous la référence SP 75 G par la société STRAHL & PITSCH, les cires microcristallines comme par exemple les cires microcristallines dont le point de fusion est supérieur à 85 °C telles que les produits HI-MIC® 1070, 1080, 1090 et 3080 commercialisés par la société NIPPON SEIRO, les cérésines ou les ozokérites comme par exemple les isoparaffines dont le point de fusion est inférieur à 40 °C telles que le produit EMW-0003 commercialisé par la société NIPPON SEIRO, les oligomères d'α-oléfine tels que les polymères PERFORMA V® 825, 103 et 260 commercialisés par la société NEW PHASE TECHNOLOGIES ; les copolymères éthylène-propylène tels que le PERFORMALENE® EP 700, les cires de polyéthylène (de préférence de poids moléculaire compris entre 400 et 600), les cires de Fischer-Tropsch, la cire de graines de tournesol commercialisée par la société KOSTER KEUNEN sous la référence sunflower wax.
On peut encore citer les cires de silicone comme les alkyl ou alkoxy-diméthicone ayant de 16 à 45 atomes de carbone, les cires fluorées.
Selon un mode de réalisation particulier, la cire utilisée dans une composition conforme à l'invention présente un point de fusion supérieur à 35 °C, mieux supérieur à 40 °C voire à 45 °C, ou encore à 55 °C.
La phase grasse des compositions conformes à l'invention peut également comprendre au moins une huile volatile ou non volatile.
On entend par huile, tout corps gras sous forme liquide à température ambiante (25 °C) et à pression atmosphérique.
Les huiles volatiles ou non volatiles peuvent être des huiles hydrocarbonées notamment d'origine animale ou végétale, des huiles synthétiques, des huiles siliconées, des huiles fluorées, ou leurs mélanges.
Au sens de la présente invention, on entend par « huile siliconée », une huile comprenant au moins un atome de silicium, et notamment au moins un groupe Si-O.
On entend par « huile hydrocarbonée », une huile contenant principalement des atomes d'hydrogène et de carbone et éventuellement des atomes d'oxygène, d'azote, de soufre et/ou de phosphore.

### Huiles non volatiles

Au sens de la présente invention, on entend par « huile non-volatile », une huile ayant une pression de vapeur inférieure à 0,13 Pa (0,01 mm de Hg).
Les huiles non volatiles peuvent notamment être choisies parmi les huiles hydrocarbonées le cas échéant fluorées et/ou les huiles siliconées non volatiles.
Comme huile hydrocarbonée non volatile convenant à la mise en oeuvre de l'invention, on peut notamment citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL; la perhydrosqualène végétale raffinée commercialisée sous la dénomination fitoderm par la société Cognis ;
- les huiles hydrocarbonées d'origine minérale ou synthétique comme par exemple :
   - les éthers de synthèse ayant de 10 à 40 atomes de carbone ;
   - les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
   - les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.
   Les esters peuvent être notamment choisis parmi les esters, notamment d'acide gras comme par exemple :
   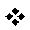 l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
   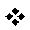 les esters de polyols, et les esters de pentaétrythritol, comme le tétrahydroxystéarate/tétraisostéarate de dipentaérythritol,
   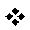 les esters de dimères diols et dimères diacides tels que les Lusplan DD-DA5® et Lusplan DD-DA7®, commercialisés par la société NIPPON FINE CHEMICAL et décrits dans la demande FR 03 02809,

   - les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
   - les acides gras supérieurs tels que l'acide oléique, l'acide linoléique, l'acide linolénique et leurs mélanges, et
   - les carbonates de di-alkyle, les 2 chaînes alkyles pouvant être identiques ou différentes, tel que le dicaprylyl carbonate commercialisé sous la dénomination Cetiol CC®, par Cognis,
   - les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,
- et leurs mélanges.

### Huiles volatiles

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).
Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.
On peut également utiliser des huiles volatiles fluorées telles que le nonafluorométhoxybutane ou le perfluorométhylcyclopentane, et leurs mélanges.
Il est également possible d'utiliser un mélange des huiles précédemment citées.

Les autres corps gras pouvant être présents dans la phase grasse sont par exemple les acides gras comportant de 8 à 30 atomes de carbone, comme l'acide stéarique, l'acide laurique, l'acide palmitique ; les alcools gras comportant de 8 à 30 atomes de carbone, comme l'alcool stéarylique, l'alcool cétylique et leurs mélanges (alcool cétéarylique).

Selon un mode de réalisation particulier, la phase grasse des compositions conformes à l'invention comprend au moins un corps gras pâteux choisi parmi les polyesters résultant de l'estérification, par un acide polycarboxylique, d'un ester d'acide hydroxy carboxylique aliphatique comprenant au moins deux groupes hydroxyle tels que définis précédemment et au moins un corps gras additionnel différent desdits polyesters. De préférence, ce corps gras additionnel est choisi parmi les huiles hydrocarbonées d'origine végétale et les huiles volatiles telles que définies ci-dessus.
Selon un mode de réalisation particulier, la composition comprend le ou les polyesters tels que définis précédemment en une quantité en matière active au moins égale à 10 % en poids, de préférence entre 10 % et 50 % en poids, et encore plus préférentiellement entre 15 et 30 % en poids du poids total de la phase grasse.

La phase grasse peut également contenir d'autres composés solubilisés dans les huiles tels que des agents gélifiants et / ou structurants.
Ces composés peuvent notamment être choisis parmi les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations « KSG » par la société Shin-Etsu, sous la dénomination « Trefil » par la société Dow Corning ou sous les dénominations « Gransil » par la société Grant Industries ; et leurs mélanges.
Ces corps gras peuvent être choisis de manière variée par l'homme du métier afin de préparer une composition ayant les propriétés, par exemple de consistance ou de texture, souhaitées.

### Phase aqueuse

La phase aqueuse de la composition selon l'invention comprend au moins de l'eau. Selon la forme galénique de la composition, la quantité de phase aqueuse peut aller de 10 à 95 % en poids, de préférence de 20 à 90 % en poids, mieux de 30 à 85 % en poids par rapport au poids total de la composition. Cette quantité dépend de la forme galénique de la composition désirée. La quantité d'eau peut représenter tout ou une partie de la phase aqueuse, et elle est généralement d'au moins 35 % en poids par rapport au poids total de la composition.
La phase aqueuse peut comprendre au moins un solvant hydrophile comme par exemple les mono-alcools inférieurs substantiellement linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le propylène glycol, le glycérol, le sorbitol, les polyéthylènes gycols et leurs dérivés, et leurs mélanges.

Selon un mode de réalisation particulier de l'invention, la composition est exempte de tensio-actif émulsionnant ou contient moins de 3 % en poids par rapport au poids total de la composition de tensio-actif émulsionnant.
Lorsqu'un tensio-actif émulsionnant est ajouté, il peut être choisi seul ou en mélange, parmi les tensio-actifs émulsionnants hydrophiles et lipophiles couramment utilisés en cosmétique. La nature du ou des tensio-actifs sera choisi selon le sens de l'émulsion, directe (H/E) ou inverse (E/H).

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des agents filmogènes ; et leurs mélanges.
Les quantités de ces différents adjuvants sont celles classiquement utilisées dans les domaines considérés. En particulier, les quantités d'adjuvants varient selon le but recherché et sont celles classiquement utilisées dans les domaines considérés, et par exemple de 0,1 à 20 %, et de préférence de 0,5 à 10 % du poids total de la composition.

### Charges et / ou pigments

Selon un mode de réalisation particulier, la composition conforme à l'invention comprend au moins une charge, de préférence à effet optique, et / ou au moins un pigment.
Au sens de la présente invention, on entend par « charge à effet optique » une charge qui permet d'obtenir des effets immédiats sur la peau, tels qu'un effet matifiant, homogénéisant, soft focus (effet de transparence, de matité, floutteur), éclaircissant.
Comme charges qui peuvent être utilisées dans la composition de l'invention, on peut citer par exemple des silices telles que le polymère de nom INCI METHYLSILANOL/SILICATE CROSSPOLYMER commercialisé sous la dénomination NLK 506 par la société TAKEMOTO OIL&FAT ; la silice telle que les microsphères de silice commercialisées sous le nom SB 700 par la société MIYOSHI KASEI ou sous les noms SUNSPHERE H-33, SUNSPHERE H-51 et SOLESPHERE H-33 par la société AGC Si-TECH ; le kaolin ; le talc ; le nitrure de bore ; les oxydes de titane comme par exemple le MICRO TITANIUM DIOXIDE MT-100 T V (nom INCI: TITANIUM DIOXIDE (and) ALUMINUM HYDROXIDE (and) STEARIC ACID) commercialisé par la société TAYCA et le MICRO TITANIUM DIOXIDE MT-100AQ (nom INCI : TITANIUM DIOXIDE (and) SILICA (and) ALUMINUM HYDROXIDE (and) ALGINIC ACID) commercialisé par la société TAYCA ; l'oxychlorure de bismuth tel que celui qui est commercialisé sour la dénomination RONAFLAIR LF 2000 par la société MERCK ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, commercialisées sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle commercialisées par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres de cellulose notamment le produit commercialisé sous la dénomination CELLULOBEADS D-10 par la société DAITO KASEI KOGYO, les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ou les dérivés d'amidon tels que le carboxyméthylamidon sodique (POMME DE TERRE) RÉTICULÉ commercialisé sous la dénomination GLYCOLYS par la société ROQUETTE. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges.
La ou les charges utilisables dans le cadre de l'invention peuvent également être choisies parmi les particules d'aérogel de silice telles que celles qui sont décrites dans les demandes de brevet WO2012/084780 et WO2012/084781.
Par "pigments", il faut comprendre des particules blanches ou colorées, minérales ou organiques, insolubles dans une solution aqueuse, destinées à colorer et/ou opacifier la composition résultante.
Comme pigments inorganiques utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome. De préférence, la composition de l'invention comprend au moins des oxydes de titane et des oxydes de fer. Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Selon un mode de réalisation particulier de l'invention, la composition comprend au moins une charge, de préférence à effet optique. De préférence, ces charges sont choisies parmi le nitrure de bore, les billes de cellulose, l'oxychlorure de bismuth et les silices.

Les charges et / ou les pigments peuvent être présents dans des quantités allant de 0 à 20 % en poids, de préférence de 0,2 à 10 % en poids, et encore plus préférentiellement de 0,5 à 5 % en poids par rapport au poids total de la composition.

### Actifs

Les compositions conformes à l'invention peuvent comprendre au moins un actif choisi parmi les dérivés C-glucoside, en particulier le C-béta-D-xylopyranoside-2-hydroxy-propane, et les dérivés d'acide cucurbique, notamment le sel de sodium de l'acide 3-hydroxy-2-pentylcyclopentyl)acétique, tels que ceux qui sont définis précédemment.

Les compositions conformes à l'invention peuvent également comprendre un ou plusieurs actifs différents des actifs choisis parmi les dérivés C-glucoside, en particulier le C-béta-D-xylopyranoside-2-hydroxy-propane, et les dérivés d'acide cucurbique, notamment le sel de sodium de l'acide 3-hydroxy-2-pentylcyclopentyl)acétique, tels que ceux qui sont définis précédemment.
On peut citer à titre d'exemple d'actif, et de façon non limitative, l'acide ascorbique et ses dérivés tels que le 5,6-di-O-diméthylsilylascorbate (vendu par la Sté Exsymol sous la référence PRO-AA), le sel de potassium du dl-alpha-tocopheryl-2l-ascorbyl-phosphate (vendu par la Société Senju Pharmaceutical sous la référence SEPIVITAL EPC), l'ascorbyl phosphate de magnésium, l'ascorbyl phosphate de sodium (vendu par la Société Roche sous la référence Stay-C 50) ; le phloroglucinol ; les enzymes ; et leurs mélanges. Parmi les actifs hydrophiles sensibles à l'oxydation, on utilise selon un mode de réalisation préféré de l'invention l'acide ascorbique. L'acide ascorbique peut être de toute nature. Ainsi, il peut être d'origine naturelle sous forme de poudre ou sous forme de jus d'orange de préférence concentré. Il peut être aussi d'origine synthétique, de préférence sous forme de poudre.
Comme autres actifs utilisables dans la composition de l'invention, on peut citer par exemple les agents hydratants tels que les hydrolysats de protéines et les polyols comme la glycérine, les glycols comme les polyéthylène glycols ; les extraits naturels ; les anti-inflammatoires ; les oligomères procyannidoliques ; les vitamines comme la vitamine A (rétinol), la vitamine E (tocophérol), la vitamine B5 (panthénol), la vitamine B3 (niacinamide), les dérivés de ces vitamines (notamment esters) et leurs mélanges ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et leurs mélanges.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention se présente sous la forme d'une émulsion. Selon un mode de réalisation particulier de l'invention, la composition se présente sous la forme d'une émulsion de type huile dans eau ou sous la forme d'une émulsion de type eau dans huile. De préférence, elle se présente sous la forme d'une émulsion de type huile dans eau.

La composition selon l'invention peut être de consistance semi-liquide du type lait par exemple, obtenues par dispersion d'une phase grasse dans une phase aqueuse, ou de suspensions ou émulsions de consistance molle, semi-solide ou solide du type crème ou baume. Ces compositions sont préparées selon les méthodes usuelles.
En outre, la composition conforme à l'invention peut être plus ou moins épaisse et avoir l'aspect d'une crème blanche ou colorée, crème liquide à épaisse, d'une pommade, d'un lait, d'un sérum, d'une pâte, d'un beurre, d'une mousse.
La composition présente de préférence un pH qui respecte la peau et qui va généralement de 3 à 8 et de préférence de 4 à 7.
Les exemples qui suivent permettront de mieux comprendre l'invention, sans toutefois présenter un caractère limitatif. Les matières premières sont nommées par leur nom INCI. Les quantités indiquées sont en % en poids en matière première, sauf mention contraire.

### Exemples

### Exemples comparatifs 1 à 3 - Emulsions huile dans eau

Les compositions A, B et C suivantes ont été préparées.

| Composition | A (invention) | B (art antérieur) | C (art antérieur) |
|---|---|---|---|
| PHASE A | | | |
| AQUA | Qsp 100 | Qsp 100 | Qsp 100 |
| Conservateur(s) | qs | qs | qs |
| GLYCERIN | 7 | 7 | 7 |

| PHASE B | | | |
|---|---|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,2 | - | 1,2 |
| HYDROXYETHYLCELLULOSE (NATROSOL 250 HHR PC de ASHLAND) | - | 1,2 | - |

| PHASE C | | | |
|---|---|---|---|
| HYDROGENATED CASTOR OIL DIMER DILINOLEATE (RISOCAST DA-L de KOKYU ALCOHOL KOGYO) | 6 | 6 | - |
| PARAFFINUM LIQUIDUM (and) CERA MICROCRISTALLINA (and) PARAFFIN (VASELINE BLANCHE CODEX 236 de AIGLON) | - | - | 6 |
| DICAPRYLYL ETHER (CETIOL OE de COGNIS) | 20 | 20 | 20 |

### Mode opératoire :

Une fois le système conservateur dissous dans le mélange eau + glycérine (à la température nécessaire), ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

Pour chacune des compositions ainsi obtenues, on évalue la stabilité immédiate à température ambiante et la stabilité après 2 mois de stockage dans des conditions de températures différentes : 4 °C, température ambiante (TA), et 45 °C.

**Stabilités :**

| | Emulsion A (invention) | Emulsion B (art antérieur) | Emulsion C (art antérieur) |
|---|---|---|---|
| Résultats à T0 | émulsion lisse, fine | Emulsion instable | Emulsion fragile qui se casse après 2 semaines de conservation |
| Après 2 mois à TA | Idem T0 Bonne Stabilité | / | / |
| Après 2 mois à 4 °C | Idem T0 Bonne Stabilité | / | / |
| Après 2 mois à 45 °C | Idem T0 | / | / |

**Propriétés cosmétiques / sensorielles :**

| | Emulsion A (invention) | Emulsion B (art antérieur) | Emulsion C (art antérieur) |
|---|---|---|---|
| Propriétés cosmétiques | Douce, lisse, homogène, brillante, laisse la peau douce, nourrie et apaisée après application | Après homogénéisation forcée : filante, hétérogène, gélatineuse | Emulsion lourde et poisseuse à l'application, laisse la peau collante et brillante |

### Exemple 4 : Crème nutritive relipidante

La composition D suivante a été préparée.

| Composition | D |
|---|---|
| PHASE A | |
| AQUA | Qsp 100 |
| Conservateur(s) | Qs |
| GLYCERIN | 7 |

| PHASE B | |
|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,2 |

| PHASE C | |
|---|---|
| HYDROGENATED CASTOR OIL DIMER DILINOLEATE (RISOCAST DA-H de KOKYU ALCOHOL KOGYO) | 6 |
| DICAPRYLYL ETHER (CETIOL OE de COGNIS) | 8 |
| SIMMONDSIA CHINENSIS OIL | 7 |
| HYDROGENATED POLYISOBUTENE (PARLEAM deNOF CORPORATION) | 5 |

### Mode opératoire :

Une fois le système conservateur dissous dans le mélange eau + glycérine (à la température nécessaire), ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

### Exemple 5 : Lait hydratant

La composition E suivante a été préparée.

| Composition | E |
|---|---|
| PHASE A | |
| AQUA | Qsp 100 |
| Conservateur(s) | Qs |
| GLYCERIN | 10 |

| PHASE B | |
|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 0,6 |

| PHASE C | |
|---|---|
| HYDROGENATED CASTOR OIL DIMER DILINOLEATE (RISOCAST DA-H de KOKYU ALCOHOL KOGYO) | 6 |
| DICAPRYLYL ETHER (CETIOL OE de COGNIS) | 10 |

### Mode opératoire :

Une fois le système conservateur dissous dans le mélange eau + glycérine (à la température nécessaire), ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

### Exemple 6 : Crème nutritive à effets immédiats

La composition F suivante a été préparée.

| Composition | F |
|---|---|
| PHASE A | |
| AQUA | Qsp 100 |
| Conservateur(s) | Qs |
| GLYCERIN | 7 |

| PHASE B | |
|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,2 |

| PHASE C | |
|---|---|
| HYDROGENATED CASTOR OIL DIMER DILINOLEATE (RISOCAST DA-H de KOKYU ALCOHOL KOGYO) | 6 |
| DICAPRYLYL ETHER (CETIOL OE de COGNIS) | 8 |
| SIMMONDSIA CHINENSIS OIL | 7 |
| HYDROGENATED POLYISOBUTENE (PARLEAM deNOF CORPORATION) | 5 |

| PHASE D | |
|---|---|
| BORON NITRIDE (SOFTOUCH BORON NITRIDE POWDER CC6058 de MOMENTIVE PERFORMANCE MATERIALS) | 2 |
| CELLULOSE (CELLULOBEADS D-10 de DAITO KASEI KOGYO) | 2 |

### Mode opératoire :

Une fois le système conservateur dissous dans le mélange eau + glycérine (à la température nécessaire), ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse. A température ambiante, ajouter la phase D.

On obtient une crème douce et fraîche à l'application. Après pénétration de la crème, la peau est nourie, confortable et son aspect est lisse et mate.

### Exemple 7

Les compositions suivantes ont été préparées.

| | Composition A (comparatif) | Composition B (invention) |
|---|---|---|
| **Phase A** | | |
| HYDROXYPROPYLTETRAHYDROPYRANTRIOL (C-béta-D-xylopyranoside-2-hydroxy-propane en solution à 30 % dans un mélange eau/1,2-propanediol 60/40 : MEXORYL SBB ® de CHIMEX) | - | 3 % MA |
| Conservateur(s) | qs | qs |
| WATER | qsp | Qsp |
| GLYCERIN | 5 | 5 |

| **PHASE B** | | |
|---|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,22 | 1,22 |

| **PHASE C** | | |
|---|---|---|
| CAPRYLIC/CAPRIC TRIGLYCERIDE | 3 | 3 |
| SIMMONDSIA CHINENSIS (JOJOBA) SEED OIL | 5 | 5 |
| CETYL ALCOHOL | 2 | 2 |
| HYDROGENATED CASTOR OIL DIMER DILINOLEATE (RISOCAST DA-L de KOKYU ALCOHOL KOGYO) | 6 | 6 |
| DICAPRYLYL ETHER (CETIOL OE de COGNIS) | 11 | 11 |

### Mode opératoire :

Une fois le système conservateur dissous dans le mélange eau + glycérine, ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

### Evaluation sensorielle :

Pour chacune des compositions A et B, on a évalué les propriétés cosmétiques selon le protocole suivant.
Les propriétés cosmétiques à l'application sont évaluées, en monadique, par un panel d'experts entraînés à la description des produits de soin. L'évaluation sensorielle des produits de soin par ce panel est réalisée comme suit : les produits sont conditionnés en pots ou flacons-pompe opaques selon la viscosité des produits. Au sein d'une même séance, les échantillons sont présentés en ordre randomisé pour chaque panéliste. 15 experts ont évalué :
- Le glissant (l'effet opposé à l'effet rêche qui induit une difficulté d'application) comme ceci : sur la main préalablement nettoyée à l'eau et au savon liquide et essuyée à l'aide d'un Kleenex, 0,05 ml de produit sont appliqués sur la moitié du dessus de la main (5 tours avec l'index et le majeur). On évalue au cours des 5 tours, et 2 minutes après application. Le descripteur "glissant" est défini comme étant la facilité d'application du produit, son potentiel à recouvrir une zone déterminée et la capacité du produit à ne pas accrocher à la peau pendant l'application. Les descripteurs sont évalués sur une échelle à 5 niveaux : Pas, Peu, Moyennement, Assez, Très.
- Le potentiel peluche : Les produits sont évalués sur une peau normale à mixte. Pour chaque produit, les experts évaluent (selon une gestuelle standardisée) le potentiel du produit à pelucher pendant l'application, puis après application et deux minutes de séchage, selon une gestuelle spécifique peluche (mouvements d'aller retour avec le dos de la main, sur la joue). La peluche est définie comme la présence de particules. La quantité de particules pouvant être « Faible », « Moyenne » ou « Importante ».
- L'effet collant pendant et après l'application. Les descripteurs sont évalués sur une échelle à 5 niveaux : Pas, Peu, Moyennement, Assez, Très.

Résultats obtenus sur un panel de 15 experts :

| Formule | A | B (invention) |
|---|---|---|
| Effet glissant | 3/15 : assez | 2/15 : assez |
| | 12/15 : très | 13/15 : très |
| potentiel peluche | Faible (13/15) | Faible (12/15) |
| | moyenne (2/15) | moyenne (3/15) |
| L'effet collant | Peu (11/15) | Peu (10/15) |
| | Pas (4/15) | Pas (5/15) |

L'évaluation comparative de ces deux formules indique que, grâce à l'utilisation de l'association d'un corps gras pâteux avec une cellulose modifiée hydrophobe, on pouvait obtenir des textures agréables bien que hautement concentrées en actifs.

### Exemple 8

Les compositions suivantes ont été préparées.

| | Composition A (invention) | Composition B (comparatif) | Composition C (comparatif) |
|---|---|---|---|
| Phase A | | | |
| WATER | Qsp 100 % | Qsp 100 % | Qsp 100 % |
| Conservateur(s) | qs | qs | qs |
| GLYCERIN | 7 | 7 | 7 |
| HYDROXYPROPYL TETRAHYDROPYRANTRIOL (C-béta-D-xylopyranoside-2-hydroxy-propane en solution à 30 % dans un mélange eau/1,2-propanediol 60/40 : MEXORYL SBB ® de CHIMEX) | 3%MA | 3%MA | 3 % MA) |

| Phase B | | | |
|---|---|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,2 | 1,2 | - |

| Phase C | | | |
|---|---|---|---|
| HYDROGENATED CASTOR OIL DIMER DILINOLEATE (RISOCAST DA-L de KOKYU ALCOHOL KOGYO) | 6 | - | 6 |
| GLYCERYL STEARATE (and) PEG-100 STEARATE (ARLACEL 165 de CRODA) | - | - | 2,5 |
| SIMMONDSIA CHINENSIS (JOJOBA) SEED OIL | 5 | 5 | 5 |
| DICAPRYLYL ETHER (CETIOL OE de COGNIS) | 15 | 21 | 15 |

### Mode opératoire :

Une fois le système conservateur dissous dans l'eau (à la température nécessaire), ajouter la phase B à la phase A sous agitation Rayneri jusqu'à homogénéisation du gel. Homogénéiser la phase C (à la température nécessaire pour avoir une phase liquide homogène). Quand les mélanges (A+B) et C sont homogènes, former l'émulsion classiquement en ajoutant C sur (A+B) sous agitation intense. Homogénéiser jusqu'à obtention d'une crème lisse.

Sur le même protocole d'évaluation défini à l'exemple 1, les résultats obtenus sur un panel de 15 experts sont présentés dans le tableau ci-dessous.

| Formule | A (invention) | Emulsion B | Emulsion C |
|---|---|---|---|
| Effet glissant | 2/15 : assez | 4/15 : pas | 10/15 : pas |
| | 13/15 : très | 11/15 : peu | 5/15 : peu |
| potentiel peluche | Faible (12/15) | Importante (11/15) | Importante (13/15) |
| | moyenne (3/15) | Moyenne (4/15) | Moyenne (2/15) |
| L'effet collant | Peu (10/15) | Très (8/15) | Très (10/15) |
| | Pas (5/15) | Assez (7/15) | Assez (5/15) |

L'évaluation comparative de ces trois formules indique que la formule selon l'invention (formule A) est moins collante, moins rêche et présente peu de peluche comparativement aux formules comparatives B et C.

### Exemple 9

Les compositions suivantes ont été préparées.

| | Emulsion A (comparative) | Emulsion B (invention) |
|---|---|---|
| PHASE A | | |
| WATER | QSP 100% | QSP 100% |
| Conservateur(s) | QS | QS |
| GLYCERIN | 7% | 7% |
| SODIUM TETRAHYDROJASMONATE (3-HYDROXY-2-PENTYLCYCLOPENTYL)ACETIC ACID, SEL DE SODIUM A 30 % DANS MELANGE EAU/DIPROPYLENE GLYCOL 70/30 A PH NEUTRE : MEXORYL SBO ® de CHIMEX) | - | 6,25 % (soit 2% MA) |

| PHASE B | | |
|---|---|---|
| CETYL HYDROXYETHYLCELLULOSE (and) | 1,2 | 1,2 |
| SODIUM NITRATE (and) SILICA (and) POLYSORBATE 60 (and) SODIUM ACETATE (POLYSURF 67 CS de ASHLAND) | | |

| PHASE C | | |
|---|---|---|
| HYDROGENATED CASTOR OIL DIMER DILINOLEATE (RISOCAST DA-L de KOKYU ALCOHOL KOGYO) | 6 | 6 |
| SIMMONDSIA CHINENSIS (JOJOBA) SEED OIL | 5 | 5 |
| DICAPRYLYL ETHER (CETIOL OE de COGNIS) | 15 | 15 |

### Mode opératoire :

Une fois le système conservateur dissous dans le mélange eau + glycérine, ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

Sur le même protocole d'évaluation défini en exemple 1, les résultats obtenus sur un panel de 15 experts figurent dans le tableau ci-dessous.

| Formule | A | B (invention) |
|---|---|---|
| Effet glissant | 4/15 : assez | 3/15 : assez |
| | 11/15 : très | 12/15 : très |
| potentiel peluche | Faible (12/15) | Faible (12/15) |
| | moyenne (3/15) | moyenne (3/15) |
| L'effet collant | Peu (11/15) | Peu (12/15) |
| | Pas (4/15) | Pas (3/15) |

L'évaluation comparative de ces deux formules indique qu'elles ont une cosméticité très proche. L'ajout du dérivé d'acide cucurbique, même à fort taux, n'affecte pas la cosméticité de la composition.

### Exemple 10

Les compositions suivantes ont été préparées.

| | Emulsion A (invention) | Emulsion B (comparatif) | Emulsion C (comparatif) |
|---|---|---|---|
| PHASE A | | | |
| WATER | QSP 100% | QSP 100% | QSP 100% |
| Conservateur(s) | QS | QS | QS |
| GLYCERIN | 7% | 7% | 7% |
| SODIUM TETRAHYDROJASMONATE (3-HYDROXY-2-PENTYLCYCLOPENTYL)ACETIC ACID, SEL DE SODIUM A 30 % DANS MELANGE EAU/DIPROPYLENE GLYCOL 70/30 A PH NEUTRE : MEXORYL SBO ® de CHIMEX) | 6,25 % (2% MA) | 6,25 % (2% MA) | 6,25 % (2% MA) |

| PHASE B | | | |
|---|---|---|---|
| CETYL HYDROXYETHYLCELLULOSE (and) SODIUM NITRATE (and) SILICA (and) POLYSORBATE 60 (and) SODIUM ACETATE (POLYSURF 67 CS de ASHLAND) | 1,2 | 1,2 | - |

| PHASE C | | | |
|---|---|---|---|
| GLYCERYL STEARATE (and) PEG-100 STEARATE (ARLACEL 165 de CRODA) | - | - | 2,5 |
| HYDROGENATED CASTOR OIL DIMER DILINOLEATE (RISOCAST DA-L de KOKYU ALCOHOL KOGYO) | 6 | - | 6 |
| SIMMONDSIA CHINENSIS (JOJOBA) SEED OIL | 5 | 5 | 5 |
| DICAPRYLYL ETHER (CETIOL OE de COGNIS) | 15 | 21 | 15 |

### Mode opératoire :

Une fois le système conservateur dissous dans le mélange eau + glycérine, ajouter la phase B à la phase A sous agitation Rayneri jusqu'à homogénéisation du gel. Homogénéiser la phase C (à la température nécessaire pour avoir une phase liquide homogène). Quand les mélanges des phases (A+B) et C sont homogènes, former l'émulsion classiquement en ajoutant la phase C sur la phase (A+B) sous agitation intense. Homogénéiser jusqu'à obtention d'une crème lisse.

Sur le même protocole d'évaluation défini en exemple 1, les résultats obtenus sur un panel de 15 experts figurent dans le tableau ci-dessous.

| Formule | A (invention) | B (comparatif) | C (comparatif) |
|---|---|---|---|
| Effet glissant | 3/15 : assez | 4/15 : pas | 10/15 : pas |
| | 12/15 : très | 11/15 : peu | 5/15 : peu |
| potentiel peluche | Faible (12/15) | Importante (11/15) | Importante (13/15) |
| | moyenne (3/15) | Moyenne (4/15) | Moyenne (2/15) |
| L'effet collant | Peu (12/15) | Très (13/15) | Très (10/15) |
| | Pas (3/15) | Assez (2/15) | Assez (5/15) |

L'évaluation comparative de ces trois formules indique que la formule selon l'invention (formule A) est moins collante, moins rêche et présente peu de peluche comparativement aux formules B et C).

## Revendications

1. Composition cosmétique sous forme d'émulsion comprenant au moins un polyester résultant de l'estérification, par un acide polycarboxylique, d'un ester d'acide hydroxy carboxylique aliphatique comprenant au moins deux groupes hydroxyle et au moins un dérivé non ionique de cellulose comprenant un ou plusieurs substituants hydrophobes ayant de 8 à 30 atomes de carbone.

2. Composition selon la revendication 1 comprenant au moins un actif choisi parmi :
1) les dérivés C-glycosides choisis parmi les composés de formule générale (I) suivante : 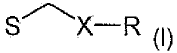 dans laquelle:
- R désigne un radical alkyle linéaire non substitué en C₁-C₄ ;
- S représente un monosaccharide choisi parmi le D-glucose, le D-xylose, la N-acétyl-D-glucosamine ou le L-fucose ;
- X représente un groupement choisi parmi -CO-, -CH(OH)-, -CH(NH₂)-;ainsi que leurs sels cosmétiquement acceptables, leurs solvates tels que les hydrates et leurs isomères optiques ; et
2) les dérivés d'acide cucurbique choisis parmi les composés de formule (II) suivante : 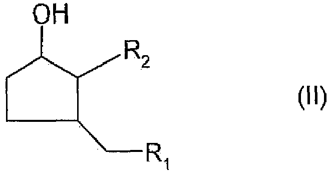 dans laquelle :
R₁ représente un radical COOR₃, R₃ désignant un atome d'hydrogène ou un radical alkyle en C₁-C₄, éventuellement substitué par un ou plusieurs groupes hydroxyle ;
R₂ représente un radical hydrocarboné, saturé ou insaturé, linéaire ayant de 1 à 18 atomes de carbones, ou ramifié ou cyclique ayant de 3 à 18 atomes de carbone ;
ainsi que leurs sels cosmétiquement acceptables, leurs solvates tels que les hydrates et leurs isomères optiques

3. Composition selon l'une quelconque des revendications 1 à 2 dans laquelle pour ledit polyester, l'acide polycarboxylique est un acide polycarboxylique aliphatique, de préférence un acide dicarboxylique aliphatique.

4. Composition selon l'une quelconque des revendications 1 à 3 dans laquelle pour ledit polyester, l'acide polycarboxylique est un dimère diacide formé à partir d'au moins un acide gras insaturé de préférence choisi parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges, encore plus préférentiellement l'acide linoléïque.

5. Composition selon l'une quelconque des revendications 1 à 4 dans laquelle pour ledit polyester, le dimère est un dimère d'un acide gras insaturé en C₈ à C₃₄, notamment en C₁₂ à C₂₂ en particulier en C₁₆ à C₂₀ et plus particulièrement en C₁₈.

6. Composition selon l'une quelconque des revendications 1 à 5 dans laquelle pour ledit polyester, le dimère diacide est saturé, c'est-à-dire qu'il ne comporte aucune double liaison carbone carbone, et qu'il est obtenu par condensation d'acide(s) gras insaturé(s) suivie éventuellement d'une hydrogénation, pour transformer les éventuelles double liaisons en simples liaisons.

7. Composition selon l'une des revendications 1 à 6 dans laquelle pour ledit polyester, l'ester d'acide hydroxy carboxylique aliphatique est choisi parmi :
a) les esters partiels ou totaux de monoacides carboxyliques aliphatiques mono hydroxylés linéaires saturés ;
b) les esters partiels ou totaux de monoacides carboxyliques aliphatiques mono hydroxylés insaturés ;
c) les esters partiels ou totaux de polyols aliphatiques en C₂ à C₁₆ ayant réagi avec un mono ou un poly acide carboxylique aliphatique mono ou poly hydroxylé ;
et leurs mélanges.

8. Composition selon l'une des revendications 1 à 7 dans laquelle pour ledit polyester, l'ester d'acide hydroxy carboxylique aliphatique est choisi parmi les esters de polyols aliphatiques en C₂ à C₁₆, lesdits polyols ayant réagi avec un acide gras aliphatique hydroxylé à chaîne saturée ou insaturée, comportant au moins 12 atomes de carbone.

9. Composition selon l'une des revendications 1 à 8 dans laquelle pour ledit polyester, l'ester d'acide hydroxy carboxylique aliphatique est choisi parmi les esters saturés ou insaturés, de polyols aliphatiques en C₂ à C₁₆, lesdits polyols ayant réagi avec l'acide ricinoléique, de préférence l'huile de ricin hydrogénée.

10. Composition cosmétique selon l'une quelconque des revendications 1 à 9 dans laquelle le ou les polyesters résultant de l'estérification, par un acide polycarboxylique, d'un ester d'acide hydroxy carboxylique aliphatique comprenant au moins deux groupes hydroxyle sont présents en quantité en matière active comprise entre 0,1 % et 30 % en poids, de préférence entre 1 et 10 %, et encore plus préférentiellement entre 2 et 8 % en poids du poids total de la composition.

11. Composition cosmétique selon l'une quelconque des revendications 1 à 10 dans laquelle le dérivé non ionique de cellulose est une hydroxyéthylcellulose substituée par un ou plusieurs substituants hydrophobes comprenant de 8 à 30 atomes de carbone, de préférence de 10 à 22 atomes de carbone, de préférence substituée par un groupement cétyle.

12. Composition cosmétique selon l'une quelconque des revendications 1 à 11 dans laquelle le degré de substitution hydrophobe va de 0,1 à 10 % en poids, de préférence de 0,1 à 1 % en poids et de manière plus préférée de 0,4 à 0,8 % en poids, du poids total du polymère.

13. Composition cosmétique selon l'une quelconque des revendications 1 à 12 dans laquelle la concentration en matière active dérivés non ioniques de cellulose à substituant(s) hydrophobe(s) dans la composition selon l'invention va de 0,05 à 20 % en poids, en particulier de 0,25 à 10 % en poids, et préférentiellement de 0,5 à 3 % en poids, par rapport au poids total de la composition.

14. Composition selon l'une quelconque des revendications 1 à 13 dans laquelle le ou les polyesters sont présents en une quantité en matière active au moins égale à 10 % en poids, de préférence entre 10 % et 50 % en poids, et encore plus préférentiellement entre 15 et 30 % en poids du poids total de la phase grasse.

15. Composition selon l'une quelconque des revendications 2 à 14 dans laquelle le dérivé C-glycoside est le C-béta-D-xylopyranoside-2-hydroxy-propane.

16. Composition selon l'une quelconque des revendications 2 et 15 dans laquelle le ou les dérivés C-glycosides sont présents en quantité en matière active comprise entre 0,03 % et 30 % en poids par rapport au poids total de la composition, en particulier entre 0,03 % et 10 % en poids, plus particulièrement entre 0,05 % et 5 % en poids.

17. Composition selon l'une quelconque des revendications 2 à 16 dans laquelle le ou les composés de formule (II) sont tels que R₁ désigne un radical choisi parmi -COOH, - COOMe, -COO-CH₂-CH₃, -COO-CH₂-CH(OH)-CH₂OH, -COOCH₂-CH₂-CH₂OH, - COOCH₂-CH(OH)-CH₃ ; et R₂ désigne un radical hydrocarboné, linéaire, saturé ou insaturé, ayant de 2 à 6 atomes de carbone.

18. Composition selon l'une quelconque des revendications 2 à 17 dans laquelle les dérivés d'acide cucurbique est le sel de sodium de l'acide 3-hydroxy 2-pentyl cyclopentane acétique.

19. Composition selon l'une quelconque des revendications 2 à 18 dans laquelle le ou les dérivés d'acide cucurbique sont présents en une teneur en matière active allant de 0,01 à 15 % en poids, par rapport au poids total de la composition, de préférence de 0,01 % à 12 % en poids, et plus particulièrement de 0,05 à 5 % en poids.

20. Composition selon l'une quelconque des revendications 1 à 19 comprenant de plus au moins une charge, de préférence choisie parmi le nitrure de bore, les billes de cellulose, l'oxychlorure de bismuth et les silices.

21. Procédé de traitement cosmétique d'une matière kératinique dans lequel on applique sur la matière kératinique une composition telle que définie à l'une quelconque des revendications 1 à 20.

## Patentansprüche

1. Kosmetische Zubereitung in Emulsionsform, welche mindestens einen Polyester, der aus der Veresterung eines mindestens zwei Hydroxygruppen umfassenden aliphatischen Hydroxycarbonsäureesters mit einer Polycarbonsäure resultiert, und mindestens ein nichtionisches Cellulosederivat umfasst, das einen oder mehrere hydrophobe Substituenten mit 8 bis 30 Kohlenstoffatomen umfasst.

2. Zubereitung nach Anspruch 1, welche mindestens einen Wirkstoff umfasst, der ausgewählt ist aus:
1) den C-Glycosidderivaten, die aus den Verbindungen der folgenden allegemeinen Formel (I) ausgewählt sind: 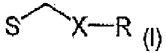 wobei:
- R ein geradkettiges unsubstituiertes C₁- bis C₄-Alkylradikal bezeichnet;
- S ein Monosaccharid darstellt, das aus D-Glucose, D-Xylose, N-Acetyl-D-Glucosamin oder L-Fucose ausgewählt ist;
- X eine Gruppe darstellt, die aus -CO-, -CH(OH)-, -CH(NH₂)- ausgewählt ist;
sowie deren kosmetisch akzeptablen Salzen, deren Solvaten wie etwa den Hydraten und deren optischen Isomeren; und
2) den Derivaten von Cucurbinsäure, die aus den Verbindungen der folgenden Formel (II) ausgewählt sind: 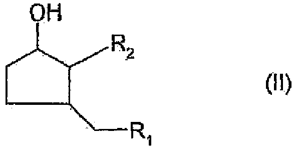 wobei:
R₁ ein Radikal COOR₃ darstellt, wobei R₃ ein Wasserstoffatom oder ein C₁- bis C₄-Alkylradikal, das eventuell mit einer oder mehreren Hydroxygruppen substituiert ist, bezeichnet;
R₁ ein gesättigtes oder ungesättigtes Kohlenwasserstoffradikal darstellt, das geradkettig mit 1 bis 18 Kohlenstoffatomen oder verzweigt oder zyklisch mit 3 bis 18 Kohlenstoffatomen ist;
sowie deren kosmetisch akzeptablen Salzen, deren Solvaten wie etwa den Hydraten und deren optischen Isomeren.

3. Zubereitung nach einem der Ansprüche 1 bis 2, wobei die Polycarbonsäure für den Polyester eine aliphatische Polycarbonsäure ist, vorzugsweise eine aliphatische Dicarbonsäure.

4. Zubereitung nach einem der Ansprüche 1 bis 3, wobei die Polycarbonsäure für den Polyester ein Disäuredimer ist, das ausgehend von mindestens einer ungesättigten Fettsäure gebildet wird, die vorzugsweise ausgewählt ist aus Undecylensäure, Linderinsäure, Myristoleinsäure, Palmitoleinsäure, Ölsäure, Linolsäure, Elaidinsäure, Gadoleinsäure, Eicosapentaensäure, Docosahexaensäure, Erukasäure, Brassidinsäure, Arachidonsäure und ihren Mischungen, noch stärker bevorzugt Linolsäure ist.

5. Zubereitung nach einem der Ansprüche 1 bis 4, wobei das Dimer für den Polyester ein Dimer einer ungesättigten C₈- bis C₃₄-, insbesondere C₁₂- bis C₂₂-, speziell C₁₆- bis C₂₀- und noch spezieller C₁₈-Fettsäure ist.

6. Zubereitung nach einem der Ansprüche 1 bis 5, wobei das Disäuredimer für den Polyester gesättigt ist, das heißt, es weist keine Kohlenstoff-Kohlenstoff-Doppelbindung auf, und es wird durch Kondensation einer ungesättigten Fettsäure (ungesättigter Fettsäuren) erhalten, eventuell gefolgt von einer Hydrierung, um die eventuellen Doppelbindungen in Einfachbindungen umzuwandeln.

7. Zubereitung nach einem der Ansprüche 1 bis 6, wobei der aliphatische Hydroxycarbonsäureester für den Polyester ausgewählt ist aus:
a) den Teilestern oder Vollestern von aliphatischen monohydroxylierten geradkettigen gesättigten Monocarbonsäuren;
b) den Teilestern oder Vollestern von aliphatischen monohydroxylierten ungesättigten Monocarbonsäuren;
c) den Teilestern oder Vollestern von aliphatischen C₂- bis C₁₆-Polyolen, die mit einer aliphatischen mono- oder polyhydroxylierten Mono-oder Polycarbonsäure reagiert haben;
und deren Mischungen.

8. Zubereitung nach einem der Ansprüche 1 bis 7, wobei der aliphatische Hydroxycarbonsäureester für den Polyester aus den Estern von aliphatischen C₂-bis C₁₆-Polyolen ausgewählt ist, wobei diese Polyole mit einer aliphatischen hydroxylierten Fettsäure mit gesättigter oder ungesättigte Kette reagiert haben, die mindestens 12 Kohlenstoffatome aufweist.

9. Zubereitung nach einem der Ansprüche 1 bis 8, wobei der aliphatische Hydroxycarbonsäureester für den Polyester aus den gesättigten oder ungesättigten Estern von aliphatischen C₂- bis C₁₆-Polyolen ausgewählt ist, wobei diese Polyole mit Ricinolsäure reagiert haben, vorzugsweise mit hydriertem Rizinusöl.

10. Kosmetische Zubereitung nach einem der Ansprüche 1 bis 9, wobei der oder die Polyester, die aus der Veresterung eines mindestens zwei Hydroxygruppen umfassenden aliphatischen Hydroxycarbonsäureesters mit einer Polycarbonsäure resultieren, in einer Wirkstoffmenge vorhanden sind, die zwischen 0,1 und 30 Gew.-%, vorzugsweise zwischen 1 und 10 Gew.-% und noch stärker bevorzugt zwischen 2 und 8 Gew.-% des Gesamtgewichts der Zubereitung liegt.

11. Kosmetische Zubereitung nach einem der Ansprüche 1 bis 10, wobei das nichtionische Cellulosederivat eine Hydroxyethylcellulose ist, die mit einem oder mehreren hydrophoben Substituenten substituiert ist, die 8 bis 30 Kohlenstoffatome, vorzugsweise 10 bis 22 Kohlenstoffatome umfassen, und vorzugsweise mit einer Cetylgruppe substituiert ist.

12. Kosmetische Zubereitung nach einem der Ansprüche 1 bis 11, wobei der Grad der hydrophoben Substitution 0,1 bis 10 Gew.-%, vorzugsweise 0,1 bis 1 Gew.-% und stärker bevorzugt 0,4 bis 0,8 Gew.-% des Gesamtgewichts des Polymers beträgt.

13. Kosmetische Zubereitung nach einem der Ansprüche 1 bis 12, wobei die Wirkstoffkonzentration der nichtionischen Cellulosederivate mit (einem) hydrophoben Substituenten in der Zubereitung gemäß der Erfindung 0,05 bis 20 Gew.-%, insbesondere 0,25 bis 10 Gew.-% und vorzugsweise 0,5 bis 3 Gew.-% beträgt, bezogen auf das Gesamtgewicht der Zubereitung.

14. Zubereitung nach einem der Ansprüche 1 bis 13, wobei der oder die Polyester in einer Wirkstoffmenge vorhanden ist (sind), die mindestens 10 Gew.-%, vorzugsweise zwischen 10 und 50 Gew.-% und noch stärker bevorzugt zwischen 15 und 30 Gew.-% des Gesamtgewichts der Fettphase beträgt.

15. Zubereitung nach einem der Ansprüche 2 bis 14, wobei das C-Glycosidderivat das C-beta-D-Xylopyranosid-2-hydroxypropan ist.

16. Zubereitung nach einem der Ansprüche 2 und 15, wobei das oder die C-Glycosidderivat(e) in einer Wirkstoffmenge vorhanden ist (sind), die zwischen 0,03 und 30 Gew. -% liegt, bezogen auf das Gesamtgewicht der Zubereitung, insbesondere zwischen 0,03 und 10 Gew.-%, spezieller zwischen 0,05 und 5 Gew.-%.

17. Zubereitung nach einem der Ansprüche 2 bis 16, wobei die Verbindung(en) der Formel (II) so beschaffen ist (sind), dass R₁ ein Radikal bezeichnet, das aus -COOH, -COOMe, -COO-CH₂-CH₃, - COO-CH₂-CH(OH)-CH₂OH, -COOCH₂-CH₂-CH₂OH, -COOCH₂-CH(OH)-CH₃ ausgewählt ist; und R₂ ein geradkettiges, gesättigtes oder ungesättigtes Kohlenwasserstoffradikal mit 2 bis 6 Kohlenstoffatomen darstellt.

18. Zubereitung nach einem der Ansprüche 2 bis 17, wobei die Derivate von Cucurbinsäure das Natriumsalz der (3-Hydroxy-2-pentylcyclopentyl)essigsäure sind.

19. Zubereitung nach einem der Ansprüche 2 bis 18, wobei das oder die Derivat (e) von Cucurbinsäure mit einem Wirkstoffgehalt vorhanden ist (sind), der 0,01 bis 15 Gew.-% beträgt, bezogen auf das Gesamtgewicht der Zubereitung, vorzugsweise 0,01 bis 12 Gew.-% und insbesondere 0,05 bis 5 Gew.-%.

20. Zubereitung nach einem der Ansprüche 1 bis 19, welche außerdem mindestens einen Füllstoff umfasst, der vorzugsweise aus Bornitrid, Cellulosekügelchen, Wismutoxychlorid und den Siliciumoxiden ausgewählt ist.

21. Verfahren zur kosmetischen Behandlung eines Keratinmaterials, bei welchem auf das Keratinmaterial eine solche Zubereitung aufgebracht wird, wie sie in einem der Ansprüche 1 bis 20 definiert ist.

## Claims

1. Cosmetic composition in the form of an emulsion comprising at least one polyester resulting from the esterification, with a polycarboxylic acid, of an aliphatic hydroxycarboxylic acid ester comprising at least two hydroxyl groups and at least one nonionic cellulose derivative comprising one or more hydrophobic substituents containing from 8 to 30 carbon atoms.

2. Composition according to Claim 1, comprising at least one active agent chosen from:
1) C-glycoside derivatives chosen from the compounds of general formula (I) below: 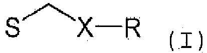 in which:
- R denotes an unsubstituted linear C₁-C₄ alkyl radical;
- S represents a monosaccharide chosen from D-glucose, D-xylose, N-acetyl-D-glucosamine and L-fucose;
- X represents a group chosen from -CO-, -CH(OH)-and -CH(NH₂)-;
and also the cosmetically acceptable salts thereof, solvates thereof such as hydrates, and optical isomers thereof; and
2) cucurbic acid derivatives chosen from the compounds of formula (II) bellow: 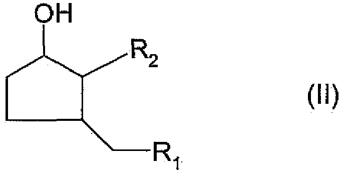 in which:
R₁ represents a radical COOR3, R3 denoting a hydrogen atom or a C1-C4 alkyl radical, optionally substituted with one or more hydroxyl groups;
R₂ represents a hydrocarbon-based radical, saturated or unsaturated, linear containing from 1 to 18 carbon atoms, or branched or cyclic containing from 3 to 18 carbon atoms;
and also the cosmetically acceptable salts thereof, solvates thereof such as hydrates, and optical isomers thereof.

3. Composition according to either one of Claims 1 and 2, in which for said polyester, the polycarboxylic acid is an aliphatic polycarboxylic acid, preferably an aliphatic dicarboxylic acid.

4. Composition according to any one of Claims 1 to 3, in which for said polyester, the polycarboxylic acid is a diacid dimer formed from at least one unsaturated fatty acid preferably chosen from undecenoic acid, linderic acid, myristoleic acid, palmitoleic acid, oleic acid, linoleic acid, elaidinic acid, gadolenoic acid, eicosapentaenoic acid, docosahexaenoic acid, erucic acid, brassidic acid and arachidonic acid, and mixtures thereof, and even more preferentially linoleic acid.

5. Composition according to any one of Claims 1 to 4, in which for said polyester, the dimer is a dimer of an unsaturated C₈ to C₃₄, especially C₁₂ to C₂₂, in particular C₁₆ to C₂₀ and more particularly C₁₈ fatty acid.

6. Composition according to any one of Claims 1 to 5, in which for said polyester, the diacid dimer is saturated, i.e. it does not comprise any carbon-carbon double bonds, and it is obtained by condensation of unsaturated fatty acid(s) optionally followed by hydrogenation, to convert any double bonds into single bonds.

7. Composition according to any one of Claims 1 to 6, in which for said polyester, the aliphatic hydroxycarboxylic acid ester is chosen from:
a) partial or total esters of saturated linear aliphatic monohydroxy monocarboxylic acids;
b) partial or total esters of unsaturated aliphatic monohydroxy monocarboxylic acids;
c) partial or total esters of C₂ to C₁₆ aliphatic polyols that have reacted with a monohydroxy or polyhydroxy aliphatic monocarboxylic or polycarboxylic acid;
and mixtures thereof.

8. Composition according to any one of Claims 1 to 7, in which for said polyester, the aliphatic hydroxycarboxylic acid ester is chosen from esters of C₂ to C₁₆ aliphatic polyols, said polyols having reacted with an aliphatic hydroxy fatty acid bearing a saturated or unsaturated chain, comprising at least 12 carbon atoms.

9. Composition according to any one of Claims 1 to 8, in which for said polyester, the aliphatic hydroxycarboxylic acid ester is chosen from saturated or unsaturated esters of C₂ to C₁₆ aliphatic polyols, said polyols having reacted with ricinoleic acid, preferably hydrogenated castor oil.

10. Cosmetic composition according to any one of Claims 1 to 9, in which the polyester(s) resulting from the esterification, with a polycarboxylic acid, of an aliphatic hydroxycarboxylic acid ester comprising at least two hydroxyl groups are present in an active material amount of between 0.1% and 30% by weight, preferably between 1% and 10% and even more preferentially between 2% and 8% by weight relative to the total weight of the composition.

11. Cosmetic composition according to any one of Claims 1 to 10, in which the nonionic cellulose derivative is a hydroxyethylcellulose substituted with one or more hydrophobic substituents comprising from 8 to 30 carbon atoms and preferably from 10 to 22 carbon atoms, preferably substituted with a cetyl group.

12. Cosmetic composition according to any one of Claims 1 to 11, in which the degree of hydrophobic substitution ranges from 0.1% to 10% by weight, preferably from 0.1% to 1% by weight and more preferably from 0.4% to 0.8% by weight relative to the total weight of the polymer.

13. Cosmetic composition according to any one of Claims 1 to 12, in which the active material concentration of the nonionic cellulose derivatives bearing (a) hydrophobic substituent(s) in the composition according to the invention ranges from 0.05% to 20% by weight, in particular from 0.25% to 10% by weight and preferentially from 0.5% to 3% by weight relative to the total weight of the composition.

14. Composition according to any one of Claims 1 to 13, in which the polyester(s) are present in an active material amount at least equal to 10% by weight, preferably between 10% and 50% by weight and even more preferentially between 15% and 30% by weight relative to the total weight of the fatty phase.

15. Composition according to any one of Claims 2 to 14, in which the C-glycoside derivative is C-beta-D-xylopyranoside-2-hydroxypropane.

16. Composition according to either of Claims 2 and 15, in which the C-glycoside derivative(s) are present in an active material amount of between 0.03% and 30% by weight relative to the total weight of the composition, in particular between 0.03% and 10% by weight and more particularly between 0.05% and 5% by weight.

17. Composition according to any one of Claims 2 to 16, in which the compound(s) of formula (II) are such that R₁ denotes a radical chosen from -COOH, -COOMe, -COO-CH2-CH3, -COO-CH2-CH(OH)-CH2OH, -COOCH2-CH2-CH2OH and - COOCH2-CH(OH)-CH3; and R₂ denotes a saturated or unsaturated linear hydrocarbon-based radical containing from 2 to 6 carbon atoms.

18. Composition according to any one of Claims 2 to 17, in which the cucurbic acid derivative is the sodium salt of 3-hydroxy-2-pentylcyclopentaneacetic acid.

19. Composition according to any one of Claims 2 to 18, in which the cucurbic acid derivative(s) are present in an active material content ranging from 0.01% to 15% by weight relative to the total weight of the composition, preferably from 0.01% to 12% by weight and more particularly from 0.05% to 5% by weight.

20. Composition according to any one of Claims 1 to 19, also comprising at least one filler, preferably chosen from boron nitride, cellulose beads, bismuth oxychloride and silicas.

21. Cosmetic process for treating a keratin material, in which a composition as defined in any one of Claims 1 to 20 is applied to the keratin material.
